# EUROPEAN PATENT APPLICATION

(11) **EP 3 753 532 A1**
(43) Date of publication of application: **23.12.2020**
(21) Application number: 19220039.2
(22) Date of filing: 30.12.2019
(51) Int. Cl.: A61F 2/24, A61F 2/962

(54) **LOADING SHEATHING CANAL AND DELIVERY SYSTEM**

(30) Priority: 21.06.2019 CN 201910541095
(71) Applicant: Lepu Medical Technology (Beijing) Co., Ltd., Beijing 102200 (CN)
(72) Inventor: LIU, Xiaojian, Beijing, Beijing 102200 (CN); WU, Yongjian, BEIJING, Beijing 100037 (CN); LIU, Tong, Beijing, Beijing 102200 (CN); QIU, Kejin, Beijing, Beijing 102200 (CN); ZHAO, Xuancheng, Beijing, Beijing 102200 (CN); CHANG, Rencao, Beijing, Beijing 102200 (CN); ZHANG, Yuxin, Beijing, Beijing 102200 (CN)
(74) Representative: Lavoix

(57) **Abstract**

The invention discloses a loading sheathing canal and a delivery system, wherein the loading sheathing canal is provided with a rigid interlayer and a flexible outer wall, and a plurality of cutting grooves are formed in the circumferential direction of the rigid interlayer, and cutting fractures are arranged between two ends of the cutting grooves, and the rigid interlayer can be directionally bent via the cutting grooves. A handle of the delivery system is connected to the loading sheathing canal for controlling the movement of the loading sheathing canal. The loading sheathing canal provided by the invention has sufficient axial tensile and compressive resistance and radial compressive resistance, and certain capability to pass through a bend, and cannot puncture a blood vessel due to insufficient bending performance.

## Description

### FIELD OF THE INVENTION

The present invention relates to the technical field of medical devices, and more particularly to a loading sheathing canal and delivery system for interventional operations.

### BACKGROUND OF THE INVENTION

With the development trend of an aging society in China, the incidence rate of senile valve degenerative diseases is increasing, among which aortic valve stenosis has gradually become the most common valvular heart disease for aging people. For patients with severe aortic valve stenosis, surgical aortic valve replacement used to be the only way to prolong life, but elderly patients are often contraindicated for such kind of surgery because of advanced age, poor physique, severe disease or other diseases. Statistics from developed countries indicate that about 1/3 of patients with severe aortic valve stenosis cannot accept conventional surgical thoracotomy because of high surgical risk or contraindications. For those patients either at high risks or with contraindications for cardiac surgery, transcatheter aortic valve implantation (TAVI) is now an effective treatment approach.

TAVI refers to the delivery of an interventional catheter through the femoral artery to deliver the prosthetic heart valve to the aortic annulus to deploy, thereby completing the implantation of the prosthetic valve and restoring the valve function. The operation does not need thoracotomy, so that the trauma is small and the postoperative recovery is fast. However, the operation of the interventional operation is very complicated, and there are many problems in the clinical practice. For example, during the delivery process, the delivery system needs to carry the valve stent to puncture from the femoral artery, then pass through the abdominal aorta, descending aorta, and pass through the aortic arch and retrograde to the root of the aorta to reach the implantation site, wherein, the delivery system forms a large bend when passing through the aortic arch; and similarly, during the replacement of the mitral valve and the tricuspid valve, the delivery system needs to pass through at least one bending part of the vessel. In addition, when the valve is not completely released at the lesion location, if the operator finds that the valve release position is not ideal enough, the operator needs to retrieve the valve to perform positioning and releasing again; when the valve is retrieved in vivo, with the absence of the assistance of auxiliary tools such as loading tools and the like, the pipe, especially the sheathing canal part for pressing and holding the valve, needs to overcome strong resistance; therefore the sheathing canal part is required to have extremely high tensile, compressive and folding resistance; at the same time, pressing and holding the sheathing canal part of the valve requires a large circuitous path through the aortic arch or the like, which requires good flexibility of the sheathing canal part.

Although the improvement of the delivery system is never stopped at present, the existing delivery system cannot well balance the axial supporting strength and the spatial three-dimensional bending performance of the loading sheathing canal, such that the loading sheathing canal cannot retrieve, relocate and release the valve due to low tensile and compressive resistance; or blood vessels are easily punctured due to insufficient bending performance of the loading sheathing canal when it passes through the aortic arch, causing great risks to clinical operations. Therefore, it is a challenge in the art to balance the two opposing properties of the sheathing canal.

### SUMMARY OF THE INVENTION

It is an object of the present invention to overcome the disadvantages of the prior art and to provide a loading sheathing canal. The sheathing canal has sufficient axial tensile and compressive resistance and radial compressive resistance, and certain capability to pass through a bend, and would not puncture blood vessels due to insufficient bending performance.

In order to achieve the above object, the embodiment of the invention provides the following technical solution:
a loading sheathing canal is provided with a rigid interlayer and a flexible outer wall, wherein a plurality of cutting grooves are formed in the circumferential direction of the rigid interlayer, and cutting fractures are arranged between two ends of the cutting grooves, and the rigid interlayer can be directionally bent via the cutting grooves.

In one embodiment, the cutting fractures of each of the cutting grooves are located on the same line.

According to one embodiment, taking the cutting groove located at an end of the rigid interlayer as the first one, the cutting fractures of the even ordinal numbers of the cutting grooves are rotated in the same direction by an angle not greater than 90°; preferably, the cutting fractures of the even ordinal numbers of the cutting grooves are all arranged to rotate 90° in the same direction; and preferably, the cutting fractures of the even ordinal numbers of the cutting grooves are all arranged to rotate 20°-60° in the same direction.

In one embodiment, the cutting fractures of each of the cutting grooves are disposed offset by an equal angle along the rigid interlayer from the top to the bottom thereof.

In one embodiment, the distal end of the rigid interlayer is provided with an expandable part comprised of a plurality of expandable structures axially secured to the distal end wherein the distal ends of each expandable structure do not restrain each other.

The expandable part may expand or contract in a radial direction of the rigid interlayer. Optionally, the expandable structure is in the shape of a meander, rectangle, circle, ellipse or notch.

In one embodiment, the rigid interlayer is further provided with a positioning hole, and the number of the positioning holes is even, and the positioning holes are arranged in axial symmetry. Optionally, the positioning holes are square, rectangular, circular or triangular in shape.

In one embodiment, the rigid interlayer is made of one or more of superelastic nickel-titanium alloy, cobalt-chrome alloy, 304 stainless steel, 316 stainless steel and 316L stainless steel, and preferably superelastic nickel-titanium alloy.

In one embodiment, the flexible outer wall is made of one or more of polyethylene, polyurethane, polyamide, and polyether block polyamide.

In one embodiment, the loading sheathing canal further comprises a lubricating layer disposed along an inner wall of the rigid interlayer, the lubricating layer being mutually fixed with the rigid interlayer and the flexible outer wall. Optionally the rigid interlayer is provided with a retention hole, and the shape of the retention hole is different from that of the positioning hole. Optionally the retention holes are used for mutually fixing the lubricating layer with the rigid interlayer and the flexible outer wall by means of hot melting and/or applying fixing glue.

In one embodiment, the lubricating layer is made of high-molecular lubricating material, optionally polytetrafluoroethylene.

It is another object of the present invention to provide a delivery system having a handle connected to the loading sheathing canal for controlling the movement of the loading sheathing canal.

In one embodiment, the diameter of a part of the distal end of the handle connected to the loading sheathing canal is smaller than the caliber of the loading sheathing canal.

Compared with the prior art, the embodiment of the invention has the following beneficial effects:
With the arrangement of a rigid interlayer, compared with the prior art the loading sheathing canal provided by the invention has the advantages that the pipe structure reinforced by the rigidity has excellent axial folding resistance and radial compressive resistance; and a plurality of cutting grooves are formed in the circumferential direction, wherein the cutting grooves are not annular closed grooves arranged along the axial direction of the rigid interlayer, but cutting fractures are arranged between the two ends of the cutting grooves; therefore, the rigid interlayer can be directionally bent along the cutting groove, a certain capability of passing through a bend thereby being achieved, and the loading sheathing canal is prevented from puncturing the blood vessel due to insufficient bending performance. In addition, due to the arrangement of the cutting fracture, the bending direction of the rigid interlayer can be controlled, and the increase of conveying difficulty caused by uncontrollable bending direction is avoided to a certain extent; and in addition, the present invention also provides a flexible outer wall on the outer side of the rigid interlayer, the flexible outer wall further reducing damage to the blood vessel during the delivery of the loading sheathing canal.

### BRIEF DESCRIPTION OF THE DRAWINGS

In order to more clearly illustrate the embodiments of the invention or technical solutions of the prior art, the drawings used in the embodiments or the description of the prior art will be briefly described below. It is obvious that the drawings in the following description are only some embodiments of the present invention, and for those skilled in the art, other drawings may also be obtained from these drawings without creative labor.
Fig. 1 is a schematic structural view of an embodiment of the present invention;
Fig. 2 is a schematic view showing the structure of a rigid interlayer provided in Embodiment 1 of the present invention;
Fig. 3 is a schematic view showing the structure of a rigid interlayer provided in Embodiment 2 of the present invention;
Fig. 4 is a schematic view showing the structure of a rigid interlayer provided in Embodiment 3 of the present invention;
Fig. 5 is an initial stage view of a loading sheathing canal recovering an implant according to one embodiment of the present invention;
Fig. 6 is a medium-term view of a loading sheathing canal recovering an implant according to one embodiment of the present invention;
Fig. 7 is a terminal stage view of a loading sheathing canal recovering an implant according to one embodiment of the present invention.

### Description of reference numerals:

1: a lubricating layer; 2: a rigid interlayer; 21: an expandable structure; 22: a retention hole; 23: a cutting groove; 231: a cutting fracture; 24: a positioning hole; 3: a flexible outer wall; and 4: an implant.

### DETAILED DESCRIPTION OF THE INVENTION

Now the technical solution of the present invention will be clearly and fully described hereinafter with reference to the accompanying drawings. It is apparent that the described embodiments are a part of the embodiments of the invention, and not all of the embodiments. All other embodiments obtained by those skilled in the art based on the embodiments of the present invention without creative efforts are within the scope of the present invention.

In describing the present invention, it is to be understood that the terms "center", "upper", "lower", "left", "right", "vertical", "horizontal", "inner", "outer", and the like, indicate orientations or positional relationships that are based on the orientations or positional relationships shown in the drawings and are merely intended to facilitate describing the present invention and simplify the description. It is not intended to indicate or imply that the referenced apparatus or element must have a particular orientation, or be constructed and operated in a particular orientation, and thus should not be construed as limiting the invention. Furthermore, the terms "first", "second", and "third" are used for descriptive purposes only and are not to be construed as indicating or implying relative importance.

In describing the present invention, it should be noted that the terms "mounted", "connected to", and "connected" are to be interpreted broadly, for example, either fixedly or removably, or integrally, unless otherwise specifically stated and defined; it can be a mechanical connection or an electrical connection; and the connection may be direct connection or indirect connection through an intermediary, and may be internal connection between two elements. It will be understood by those of ordinary skill in the art that the specific meanings of the above terms in the present invention may be specifically understood.

Furthermore, the technical features involved in different embodiments of the invention described below may be combined with each other as long as they do not conflict with each other.

### Embodiment 1

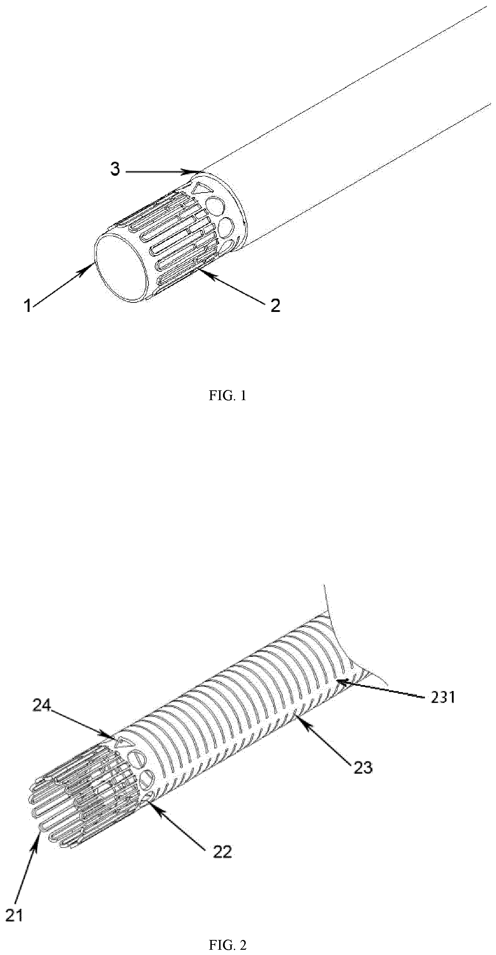

As shown in Fig. 1, the embodiment provides a loading sheathing canal having a rigid interlayer 2 and a flexible outer wall 3, wherein the rigid interlayer 2 is provided with a plurality of cutting grooves 23 in the circumferential direction; and the cutting groove 23 is not closed along the circumferential direction of the rigid interlayer 2, but it is provided with a cutting fracture 231 between two ends of the cutting groove 23. The rigid interlayer 2 can be directionally bent by means of the cutting groove 23.

Compared with the prior art, the loading sheathing canal of the present invention has the advantages that due to the fact that the rigid interlayer 2 is arranged, the pipe structure reinforced by rigidity has excellent axial folding resistance and radial compression resistance. A plurality of cutting grooves 23 are formed in the circumferential direction of the rigid interlayer 2, wherein the cutting groove 23 is not annular closed groove arranged in the axial direction of the rigid interlayer 2, but a cutting fracture 231 is arranged between the two ends of the cutting groove 23 instead so that the rigid interlayer 2 can be directionally bent along the cutting groove 23, thereby certain bending capability being achieved. The loading sheathing canal is prevented from puncturing a blood vessel due to insufficient bending performance. In addition, due to the arrangement of the cutting fracture 231, the bending direction of the rigid interlayer 2 can be controlled, causing the increase of conveying difficulty led by uncontrollable bending direction to be avoided to a certain extent; and in addition to this, the present invention also provides a flexible outer wall 3 on the outer side of the rigid interlayer 2, the flexible outer wall 3 further reducing damage to the blood vessel during the delivery of the loading sheathing canal.

In some embodiments, the rigid interlayer 2 can be made of superelastic nickel-titanium alloy, cobalt-chromium alloy, 304 stainless steel, 316 stainless steel, or 316L stainless steel. A metal skeleton is formed by cutting metal tubes, thereby providing the loading sheathing canal with a certain radial and axial support force. In interventional operations, implanted devices can be pre-compressed into the loading sheathing canal, and stay in compression state under the pressure of the loading sheathing canal. After the implanted device is delivered to the implantation site, the implanted device can be exported by a delivery system to be released.

As shown in Fig. 2, the cutting edge on the loading sheathing canal may be formed of a variety of cutting shapes, including mainly an expandable structure 21, a retention hole 22, a cutting groove 23, and a positioning hole 24. In some embodiments, the cutting groove 23 is arranged in circumferential direction of the rigid interlayer 2 and can be through groove or blind groove, wherein a through groove is arranged. The head and tail of each cutting groove 23 are not connected, and the cutting fracture 231 is arranged between the ports of the head and the tail, and the cutting fractures 231 of each cutting groove 23 are located on the same line. In some embodiments, the cutting fractures 231 of each cutting groove 23 have the same length, and the center of each cutting fracture 231 is located on the same line. The cutting groove 23 can bend the rigid interlayer 2 in the loading sheathing canal, and the cutting fracture 231 can ensure the connection strength of the rigid interlayer 2 when the rigid interlayer 2 is bent and limit the bending direction of the rigid interlayer 2 at the same time. As shown in FIG. 2, the cutting fracture 231 is correspondingly designed along the same line to facilitate the upward and downward bending of the catheter. Meanwhile, due to the design, the rigid interlayer 2 can be bent to the maximum extent, and the capability of passing through a bend is increased.

A plurality of expandable structures 21 are provided, wherein the proximal end of each expandable structure 21 is axially fixedly connected with or integrally formed with the distal end of the rigid interlayer 2 without mutual restraint between the distal ends of each expandable structure 21. Each expandable structure 21 forms an expandable part arranged at the distal end of the loading sheathing canal, and the expandable part can be expanded or contracted in the radial direction of the rigid interlayer 2 so as to increase the deformation quantity of the distal end of the loading sheathing canal. Since there is no mutual restraint between the distal ends of each expandable structure 21, the distal ends of the expandable parts can freely expand when the expandable parts are stressed. The proximal ends of the expandable structure 21 can impart a certain amount of restraining force to the expandable parts, thereby reducing the deformation of the expandable part. Therefore, during the process of the implant being recovered into the loading sheathing canal, the distal end of the rigid interlayer 2 will form a horn shape that facilitates the recovery of the implant due to differences in stress state and deformation quantity.

The expandable structure 21 can have a rectangular shape, a circular shape, an oval shape, a notch shape and other shapes facilitating the expansion of the pipes. Of course, the width of the rod of the expandable structure 21 can be adjusted according to different requirements. When the width of the rod is wide, the deformation can be reduced and the restraining force can be increased; and when the width of the rod is narrow, the deformability can be increased, facilitating the recovery of the implant into the loading sheathing canal, and at the same time, the restraining force will be small.

In addition, the rigid interlayer 2 of the present invention is provided with a retention hole 22 and a positioning hole 24 respectively, and the purpose of arranging the retention hole 22 is to fix the rigid interlayer 2 and the flexible outer wall 3 to each other. Of course, in order to avoid damage caused by frictions easily occurred when the implant enters and exits the loading sheathing canal, a lubricating layer 1 can be attached to the inner wall of the rigid interlayer 2. The lubricating layer 1 is preferably made of a high-molecular lubricating material, such as polytetrafluoroethylene, so as to reduce the friction between the loading sheathing canal and the implant and facilitate the release and recovery of the implant device. At this time, the retention hole 22 is used for fixing the lubricating layer 1, the rigid interlayer 2 and the flexible outer wall 3 to each other by means of hot melting or applying fixing glue or the like. Wherein, the retention hole 22 is preferably uniformly arranged in the circumferential direction of the rigid interlayer 2. The shape of the retention hole 22 may be square, circular, triangular, rectangular or other shapes.

In order to facilitate directional positioning of the delivery sheathing canal in an interventional operation, the delivery sheathing canal can also be provided with a positioning hole 24, wherein the number of the positioning hole 24 is preferably set to be even and the positioning hole 24 is arranged in axial symmetry on the delivery sheathing canal. In use, a developer is infused into the delivery sheathing canal, and the developer causes the positioning hole 24 to develop more clearly under X-rays. When the positioning hole 24 is not substantially shown on the developing equipment, proper placement of the delivery sheathing canal is indicated and deeply implant the delivery catheter can be continued. It will be apparent that the shape of the positioning hole 24 may be square, circular, triangular, rectangular, etc. to facilitate the convenience for development recognition.

It should be noted, however, that the shapes of the positioning hole 24 and the retention hole 22 must be different. For example, when the positioning hole is triangular, the retention hole is circular, and both the positioning hole 24 and the retention hole 22 can serve as developing holes to guide interventional operations.

### Embodiment 2

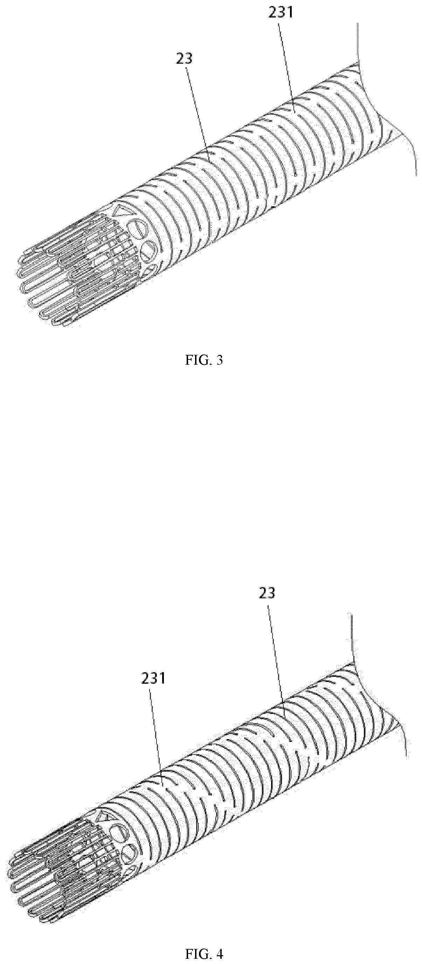

Unlike Embodiment 1, referring to Fig. 3, in this embodiment taking the cutting groove 23 located at the end of the rigid interlayer 2 as the first one, the cutting fractures 231 of the even ordinal numbers of the cutting grooves 23 are rotated in the same direction by an angle not greater than 90°; in one embodiment, the cutting fractures 231 of the even ordinal numbers of the cutting grooves 23 are all arranged to rotate 90° in the same direction; and in one embodiment, the cutting fractures of the even ordinal numbers of the cutting grooves are all arranged to rotate 20°-60° in the same direction. The design makes it possible to achieve a large-angle bending of the loading sheathing canal in four dimensions perpendicular to each other and to increase the bending direction of the catheter, wherein the maximum-angle bending can be achieved in four dimensions perpendicular to each other when the cutting fractures 231 of the even ordinal numbers of the cutting grooves 23 are all arranged to rotate 90° in the same direction, but the bending angle is limited in comparison with Embodiment 1.

### Embodiment 3

Unlike Embodiment 1, referring to Fig. 4, in this embodiment the cutting fractures 231 of each cutting groove 23 are disposed offset by an equal angle, typically no more than 5°, along the rigid interlayer 2 from the top to the bottom thereof. The design ensures that the catheter is free to bend in any direction such that it can be adjusted at any angle during the implantation. However, like the second embodiment, this circumferential design also limits the bending angle of the loading sheathing canal to some extent compared to the embodiment.

### Embodiment 4

The present invention also provides a delivery system (not shown) having a handle connected to the loading sheathing canal for controlling the movement of the loading sheathing canal. The proximal end of the loading sheathing canal can be connected with a handle pipe of which the caliber is smaller than that of the loading sheathing canal, and the overall catheter is controlled by a control handle.

The invention will now be further explained in connection with a heart valve stent 4.

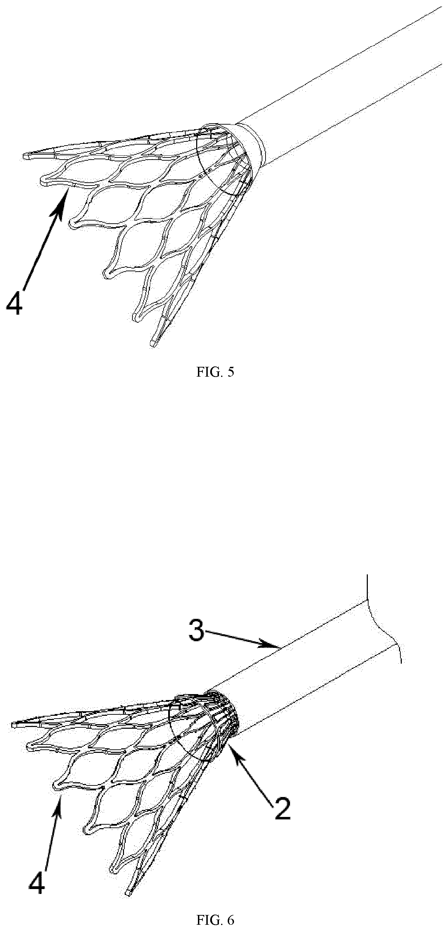

Fig. 5 shows a simulated view of the recovery of an aortic valve stent 4 formed by laser cutting a tube of superelastic nickel-titanium alloy material and implanted into the aortic valve position of human body by self-expandable, wherein a prosthetic valve (not shown) is fixed inside the stent 4. The proximal end of the aortic valve stent 4 is connected to a delivery system and can be delivered in the circulatory system of a human body via a loading sheathing canal.

During operation, the aortic valve replacement device is first loaded into the delivery system, and the stent 4 is retracted into the loading sheathing canal and maintains a collapsed state in a dense tubular configuration while not being released. After the aortic valve replacement device is loaded, the delivery system along with the aortic valve replacement device is delivered to the valve implantation site along the circulatory system. Valve replacement is typically performed by puncturing from the femoral artery, passing through the abdominal aorta and the descending aorta, and then passing through the aortic arch, and retrograding to the aortic valve site at the aortic root. Because the vascular system of the human body is tortuous and complicated, good compliance of the catheter itself is required so that the bending direction can be adjusted adaptively to conduct the forward delivery along the vascular system as the loading sheathing canal is usually advanced along the vessel wall. Because the bending angle of the aortic arch part is large, the loading sheathing canal needs to be positioned at the moment. The positions of the positioning hole 24 and the retention hole 22 are observed via a developing equipment so that the bending direction of the loading sheathing canal is ensured to be consistent with the bending direction of the aortic arch. When the loading sheathing canal passes through the aortic arch, the distal end of the loading sheathing canal is firstly close to the vessel wall and is bent due to the compression of the vessel wall. The bending angle of the loading sheathing canal becomes larger and larger with the delivery of the aortic valve, and finally the loading sheathing canal smoothly enters the aortic valve replacement site through the aortic arch.

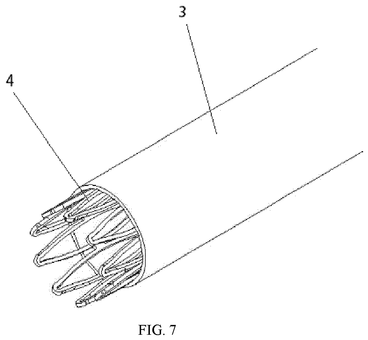

As shown in Fig. 6, when the aortic valve replacement device is delivered to the replacement site, the stent 4 is released, and the stent 4 expands and fixes the original aortic valve position. When the valve needs to be recovered and repositioned due to improper position, severe regurgitation, wrong size selection and the like after the stent 4 is released, the stent 4 needs to be repositioned or recovered. The handle controlling the delivery system retracts the stent 4; when the stent 4 retracts, the proximal end of the stent 4 is firstly stressed and compressed. As the expandable structure 21 of the rigid interlayer 2 in the loading sheathing canal is stressed and expanded, the inner diameter of the distal end of the loading sheathing canal is enlarged and the stent recovery resistance is reduced, so that the stent 4 can be relatively easily retracted into the loading sheathing canal as shown in Fig. 7.

The above description is only the specific embodiment of the present invention, but the scope of the present invention is not limited thereto. Any change or replacement that can be easily conceived by those skilled in the art within the technical scope disclosed by the present invention should be covered by the scope of the present invention. Therefore, the scope of the invention should be determined by the scope of the appended claims.

## Claims

1. A loading sheathing canal, **characterized by** comprising a rigid interlayer and a flexible outer wall, wherein a plurality of cutting grooves are formed in the circumferential direction of the rigid interlayer; a cutting fracture is arranged between two ends of the cutting groove, and the rigid interlayer can be directionally bent via the cutting groove.

2. The loading sheathing canal of claim 1, **characterized in that** the cutting fractures of each cutting groove are on a same line.

3. The loading sheathing canal according to claim 1, **characterized in that**, taking the cutting groove located at an end of the rigid interlayer as the first one, the cutting fractures of the even ordinal numbers of the cutting grooves are rotated in the same direction by an angle not greater than 90°; preferably, the cutting fractures of the even ordinal numbers of the cutting grooves are all arranged to rotate 90° in the same direction; and preferably, the cutting fractures of the even ordinal numbers of the cutting grooves are all arranged to rotate 20°-60° in the same direction.

4. The loading sheathing canal of claim 1, **characterized in that** the cutting fractures of each cutting groove are disposed offset by an equal angle along the rigid interlayer from top to bottom thereof.

5. The loading sheathing canal of any one of claims 1-4, **characterized in that** a distal end of the rigid interlayer is provided with an expandable part wherein the expandable part consists of a plurality of expandable structures axially secured to the distal end and the distal ends of each expandable structure do not restrain each other.

6. The loading sheathing canal of claim 5, **characterized in that** the expandable part is expandable or contractible in a radial direction of the rigid interlayer, wherein optionally the expandable structure is in the shape of a meander, rectangle, circle, ellipse or notch.

7. The loading sheathing canal of any one of claims 1 to 4, or 6, **characterized in that** the rigid interlayer is provided with positioning holes, wherein the number of the positioning holes is even and the positioning holes are arranged in axial symmetry, and optionally the positioning holes are square, rectangular, circular or triangular in shape.

8. The loading sheathing canal of claim 1, **characterized in that** the rigid interlayer is made of one or more of superelastic nickel-titanium alloy, cobalt-chromium alloy, 304 stainless steel, 316 stainless steel, and 316L stainless steel, and preferably superelastic nickel-titanium alloy.

9. The loading sheathing canal of claim 1, **characterized in that** the flexible outer wall is made of one or more of polyethylene, polyurethane, polyamide, and polyether block polyamide.

10. The loading sheathing canal of claim 1, **characterized in that** the loading sheathing canal further comprises a lubricating layer arranged along the inner wall of the rigid interlayer, and the lubricating layer is mutually fixed with the rigid interlayer and the flexible outer wall.

11. The loading sheathing canal of any one of claims 1-4, 6, 8-10, **characterized in that** the rigid interlayer is further provided with a retention hole, wherein the number of the retention holes is even, and the shape of the retention holes arranged in axial symmetry is different from that of the positioning holes; and optionally the retention holes are used for mutually fixing the lubricating layer with the rigid interlayer and the flexible outer wall by means of hot melting and/or applying fixing glue.

12. The loading sheathing canal of claim 10, **characterized in that** the lubricating layer is made of a high molecular lubricating material, optionally polytetrafluoroethylene.

13. A delivery system, **characterized in that** a handle of the delivery system is connected to the loading sheathing canal of any one of claims 1-12, the handle being used to control the movement of the loading sheathing canal.

14. The loading sheathing canal of claim 13, **characterized in that** a proximal end of the loading sheathing canal is connected to the handle by a catheter, wherein the catheter has a diameter smaller than the caliber of the loading sheathing canal.
